# EUROPÄISCHE PATENTSCHRIFT

(11) **EP 0 908 159 B1**
(45) Veröffentlichungstag und Bekanntmachung des Hinweises auf die Patenterteilung: **09.06.2004**
(21) Anmeldenummer: 98116720.8
(22) Anmeldetag: 03.09.1998
(51) Int. Cl.: A61F 2/36

(54) **Oberschenkelprothese**
Femoral prosthesis
Prothèse fémorale

(30) Priorität: 16.09.1997 DE 19740689
(43) Veröffentlichungstag der Anmeldung: 14.04.1999
(73) Patentinhaber: Copf, Franz, D-70178 Stuttgart (DE)
(72) Erfinder: Copf, Franz, D-70178 Stuttgart (DE)
(74) Vertreter: Ostertag, Reinhard

(56) Entgegenhaltungen:
- EP-A- 0 319 171
- EP-A- 0 672 396
- WO-A-97/36558
- DE-A- 3 928 845
- DE-A- 19 610 729
- DE-U- 9 402 934
- FR-A- 2 676 914
- US-A- 4 678 471
- US-A- 4 938 771

## Beschreibung

Die Erfindung betrifft eine Oberschenkelprothese gemäß dem Oberbegriff des Anspruches 1.

Eine Oberschenkelprothese ist in der DE 36 16 655 A1 offenbart. Sie hat einen aus von der Tragplatte herabhängenden Verankerungspfeilern gebildeten Verankerungsschaft. Die Verankerungspfeiler tragen jeweils eine Vielzahl axial aufeinanderfolgender ringförmiger Verankerungsbunde. In die Zwischenräume zwischen den einzelnen Verankerungspfeilern kann Spongiosa einwachsen. Auf diese Weise ist die Prothese nach Einheilung ohne Zement fest mit dem Femur verbunden.

Um die Prothese unterschiedlichen großen Kavitäten im proximalen Ende des Femurs anpassen zu können, kann man freie Enden von Verankerungspfeilern je nach Bedarf kürzen. Dies ist jedoch nur bei frei auslaufenden Verankerungspfeilern ohne Schwierigkeiten möglich. Bei untereinander verbundenen Verankerungspfeilern wäre ein Heraustrennen von Teilen der Käfigstruktur mit einem so starken und für den Chirurgen nicht in seinen Konsequenzen überschaubaren Eingriff in die mechanisch tragende Struktur verbunden, daß die Haltbarkeit und Tragfähigkeit der Prothese nicht mehr gewährleistet wäre. Auch bei Schaftprothesen, die unter Verwendung von Zement mit der Corticalis des Femurs verbunden werden, ist es offensichtlich mit großen Schwierigkeiten verbunden, durch mechanische Nachbearbeitung der Prothese im Verlaufe der Operation eine bessere Anpassung an die Knochengeometrie zu erzielen. Insbesondere bei zementierten Prothesen wäre es aber wünschenswert, daß die Zementschicht lokal nicht zu stark variiert, was bedeutet, daß der Verankerungsabschnitt schon von Hause aus der Knochengeometrie nahekommen muß.

Eine Oberschenkelprothese gemäß dem Oberbegriff des Anspruchs 1 ist aus US-A-4 938 771 bekannt. In der US-A-4 938 771 ist eine ebenfalls weit ins Innere des Femurs reichende Prothese offenbart, deren Schaft mit großem Krümmungsradius bogenförmig gekrümmt ist. Ein oberer Abschnitt des Prothesenschaftes, der dem Endabschnitt des Femurs entspricht, ist als Käfigstruktur ausgebildet.

In der FR-A-2 676 914 ist eine zementierte Prothese offenbart, die große Länge aufweist und sich bis in den mittleren Abschnitt des den Femur bildenden Röhrenknochens hineinerstreckt. Diese Prothese ist in Form eines sehr flachen S geschwungen, um der Geometrie des Knochenkanales Rechnung zu tragen. Im untersten Abschnitt hat der Prothesenschaft die Form eines Zylinders, der mit Abstand vom distalen Schaftende zunehmend abgeplattet ist, so daß man zwei planparallele Hauptflächen erhält, die ventral bzw. dorsal liegen. Die Schaftquerschnittsfläche ist in der oberen Prothesenhälfte progressiv um die Schaftlängsachse verdreht.

In der DE-A-39 28 845 ist eine zementfrei zu implantierende Prothese offenbart, welche eine das resezierte obere Ende des Femures überdeckende Tragplatte aufweist, deren Unterseite eine Vielzahl von axial beabstandete Verankerungsbunde aufweisenden Verankerungspfeilern trägt.

In der DE-U-94 02 934 ist ein Prothesenteil offenbart, das demjenigen nach der FR-A-2 676 914 ähnelt, jedoch nicht in Form eines flachen S sondern kreisbogenförmig verläuft. Auch dieses bekannte Prothesenteil reicht weit ins Innere des Femurs.

Auch ein in der EP-A-0 319 171 offenbartes Prothesenteil hat einen langgestreckten Schaft, der tief in den Femur eingesetzt wird.

Gleiches gilt für ein Prothesenteil, wie es in der EP-A-0 672 396 gezeigt ist. Dabei hat dieses Prothesenteil einen Knick zwischen einem oberen Schaftabschnitt, der insgesamt dem Endabschnitt des Femurs entspricht, und einem stabförmigen unteren Schaftabschnitt, der dem diaphysären Röhrenabschnitt des Femurs zugeordnet ist.

In der US-A-4 678 471 ist eine Oberschenkelprothese offenbart, die einen abgeknickten, in eine Bohrung des Oberschenkelknochens einzementierbaren Schaft aufweist. Der Knickwinkel des Schaftes ist so gewählt, daß die Kugel der Oberschenkelprothese an die Stelle der ausgefallenen körpereigenen Gelenkkugel zu liegen kommt.
An der Übergangsstelle zwischen Gelenkkugel und Prothesenschaft ist ein ringförmiger Flansch vorgesehen, der senkrecht auf dem Schaftende steht und im implantierten Zustand über der Resektionsfläche liegt.

In der gemäß Artikel 54 (3) und (4) EPÜ zu berücksichtigenden WO-A- 97/36558 ist eine zementierbare Prothese beschrieben, die einen Schaft aufweist, der die Form einer um ihre Längsachse tordierten Pyramide hat. An der Übergangsstelle zwischen dem Schaft und einem Montagezapfen für eine Gelenkkugel ist eine Tragplatte vorgesehen, welche das obere Ende des resezierten Femurs überdeckt.

Durch die vorliegende Erfindung soll daher eine Oberschenkelprothese gemäß dem Oberbegriff des Anspruches 1 so weitergebildet werden, daß sie ohne mechanische Nachbearbeitung besser der Geometrie der Corticalis im oberen Endschaft des Femurs entspricht.

Diese Aufgabe ist erfindungsgemäß gelöst durch eine Oberschenkelprothese mit den im Anspruch 1 angegebenen Merkmalen.

Es wurde erkannt, daß man dadurch, daß man den Schaft der Prothese aus zwei gegeneinander verkippten Schaftabschnitten aufbaut, generell der Geometrie der Corticalis im oberen Endabschnitt des Femurs besser Rechnung tragen kann.

Mit einem Satz von im übrigen gleiche Abmessungen aufweisenden Prothesen, die sich bezüglich des Knickwinkels zwischen den beiden Schaftabschnitten unterscheiden, kann man einer sehr großen Anzahl unterschiedlicher Implantierungsbedingungen Rechnung tragen.

Damit ist es nicht mehr notwendig, die Fehlanpassung der Prothesenaußenfläche an die Innenfläche der Corticalis über lokal stark unterschiedliche Dicke aufweisende Zementschichten zu kompensieren.

Vorteilhafte Weiterbildungen der Erfindung sind in Unteransprüchen angegeben.

Die im Anspruch 2 angegebenen Winkel sind für die am häufigsten angetroffenen Oberschenkelgeometrien am geeignetsten. Stuft man den Knickwinkel in einem Prothesensatz in Abständen von 5°, so ist in dem Prothesensatz immer eine, die unter nur geringen Restdickenschwankungen der Zementschicht der Geometrie des oberen Endes des Oberschenkels gut angepaßt ist.

Anspruch 3 gibt bevorzugte Werte für den Abstand der Schaft-Knickstelle vom distalen Ende des Schaftes an. Wiederum kann man in einem Prothesensatz Prothesen vorsehen, bei welchen die Schaft-Knicklinie unterschiedlichen Abstand vom distalen Ende des Schaftes aufweist, wobei die Stufung vorzugsweise in Abständen von 5 mm erfolgt.

Bei einer Oberschenkelprothese gemäß Anspruch 4 verläuft die Tragplatte bei implantierter Prothese im wesentlichen in horizontaler Richtung.

Mit der im Anspruch 5 angegebenen Querschnittsform für die Schaftabschnitte erhält man eine besonders gute Anpassung an den transversalen Querschnitt der Kavität, die durch Entfernen von Spongiosa aus dem oberen Endabschnitt des Femurs erhalten wird. Dabei erlaubt die im Anspruch 6 ebenfalls angesprochene Torsion mindestens eines Unterabschnittes mindestens eines der Schaftabschnitte eine besonders gute Anpassung an die Innenfläche der Corticalis.

Bevorzugt verwendete Torsionswinkel eines solchen Schaftabschnitt-Unterabschnittes sind im Anspruch 6 angegeben.

Auch die Weiterbildung der Erfindung gemäß Anspruch 7 ist im Hinblick darauf von Vorteil, daß die Zementschicht zwischen Außenfläche des Schaftes und Innenfläche der Corticalis im wesentlichen konstante Dicke hat.

Mit der Weiterbildung der Erfindung gemäß Anspruch 8 wird erreicht, daß der zum Implantieren verwendete Zement besonders gut an der Schaftaußenseite haftet.

Die Weiterbildung der Erfindung gemäß Anspruch 9 ermöglicht es, den Trochander major des Femurs nur teilweise zu resezieren und teilweise stehenzulassen. Dies ist im Hinblick auf gute Übertragung von transversalen Kräfte von Prothese auf die Corticalis von Vorteil.

Die Weiterbildung der Erfindung gemäß Anspruch 10 ist im Hinblick auf hohe mechanische Belastbarkeit der Prothese und im Hinblick auf niedere Herstellungskosten von Vorteil.

Nachstehend wird die Erfindnung anhand eines Ausführungsbeispiels unter Bezugnahme auf die beiliegende Zeichnung näher erläutert. In dieser zeigen:
- Figur 1:: eine Ansicht einer Oberschenkelprothese gesehen in Richtung von ventral nach dorsal, wobei eine Gelenkkugel und ein oberer Abschnitt eines Femurs, in welchen die Prothese implantiert ist, schematisch angedeutet sind;
- Figur 2:: eine Ansicht der in Figur 1 gezeigten Prothese in Richtung von medial nach lateral, also in Figur 1 links nach rechts;

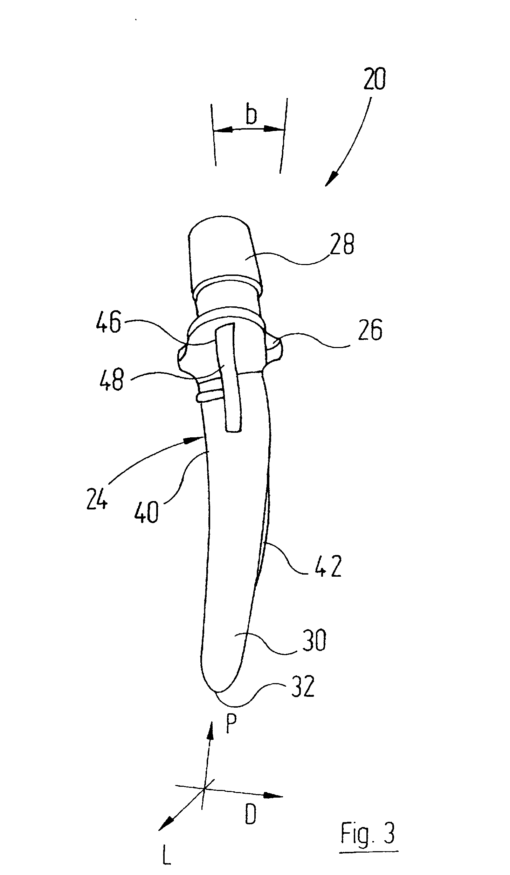

- Figur 3:: eine Ansicht der Prothese nach Figur 1 in Richtung von lateral nach medial, also in Figur 1 rechts nach links; und
- Figur 4:: eine Ansicht der Prothese nach Figur 1 in Richtung von distal nach proximal, also in Figur 1 von unten.

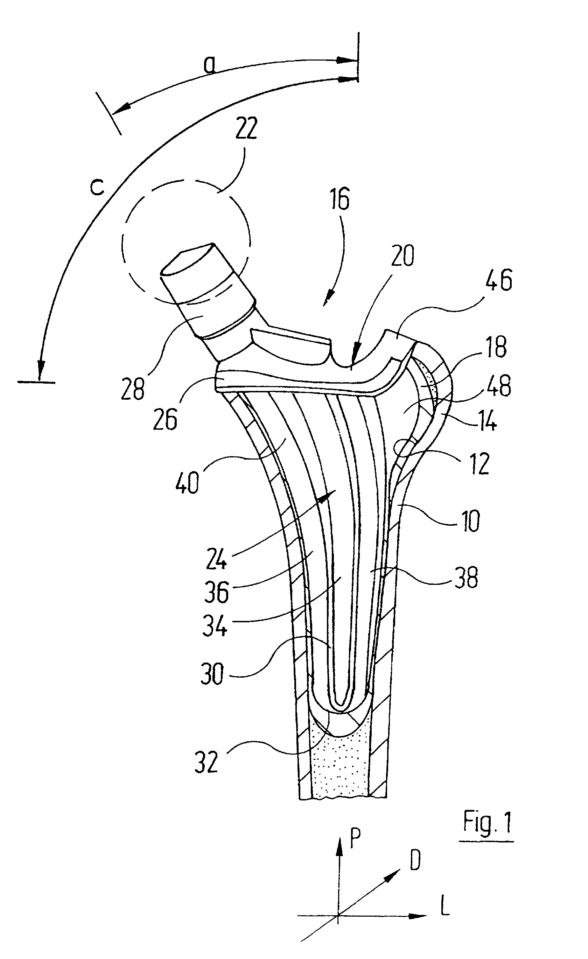

In der Zeichnung ist bei 10 proximale Teil eines Femurs wiedergegeben. Der Trochander minor und die von ihm getragene Gelenkkugel sind reseziert, wobei die Resektionslinie im wesentlichen in horizontaler Richtung verläuft. Ein in Figur 1 rechts gelegener Endabschnitt der Resektionslinie steigt nach oben an, so daß ein Teil des Trochander major erhalten bleibt.

Aus dem oberen Endabschnitt des Femurs 10 ist die Spongiosa ausgeräumt, so daß man eine Kavität 12 erhält, die durch die Innenfläche der stehenbleibenden Corticalis 14 begrenzt ist.

In die Kavität 12 ist eine Prothese 16 über eine Zementschicht 18 fest eingesetzt. Die Prothese 16 besteht aus einem Titan geschmiedeten Prothesenkörper 18, der nachstehend noch in Einzelheiten beschrieben werden wird, sowie einer aus Keramik gefertigten vom Prothesenkörper 20 getragenen Gelenkkugel aus Keramik mit polierter Oberfläche.

Der Prothesenkörper 20 besteht aus drei Hauptelementen, nämlich einem in der Kavität 12 liegenden Schaft 24, einer das obere Ende der resezierten Corticalis 14 im wesentlichen bündig abdeckenden Tragplatte 26 sowie einem von der letzteren getragenen Tragzapfen 28, auf welchen die Gelenkkugel 22 angebracht ist.

Zur Beschreibung der Geometrie des Pothesenkörpers 20 werden nachstehend und in den Ansprüchen dem Prothesenkörper eigene Koordinaten verwendet. Dabei wird ausgegangen von der Zeichenebene von Figur 1, die in allerdings sehr grober Näherung auch eine Art Mittelebene des Prothesenkörpers darstellt. Aus diesen Gründen wird für diese Ebene die Bezeichnung Hauptebene gewählt.

Die Längsachse des unteresten Endabschnittes des Schaftes 24, die in der Hauptebene liegt, wird nachstehend als Proximalachse bezeichnet. Sie verläuft in Figur 1 von unten nach oben, also bezogen auf das Ende des Femurs von distal nach proximal. Diese Achse ist in der Zeichnung mit P bezeichnet. Eine weitere Achse, die in der Zeichnung von links nach rechts verläuft, also bezogen auf das Femurende von medial nach lateral, wird nachstehend als Lateralachse bezeichnet und ist in der Zeichnung bei L dargestellt. Die dritte Achse des zur Beschreibung der Geometrie des Prothesenkörpers 20 verwendeten kartesischen Koordinatensystemes verläuft senkrecht zur Zeichenebene von Figur 1, also von ventral nach dorsal. Diese Achse wird nachstehend als D-Achse bezeichnet und trägt das Bezugszeichen D. Die Hauptebene entspricht somit der L-P-Ebene.

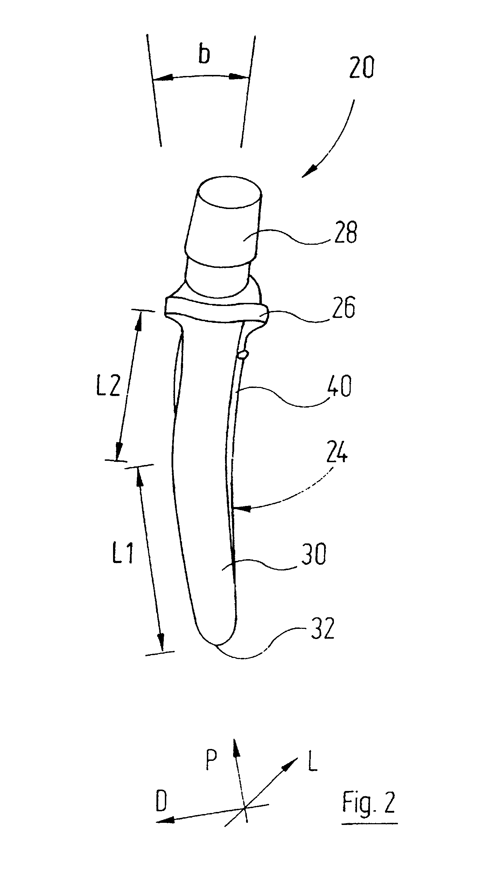

Der Zusammenhang zwischen den oben angegebenen protheseneigenen Koordinaten und der sogenannten physiologischen Längsache (PLA) des (gesamten) Oberschenkelknochens ist folgender: Die PLA fällt im wesentlichen mit der P-Achse zusammen. In der PL-Ebene (Zeichenebene von Figur 1) ist die Projektion der PLA um 1,2° im Uhrzeigersinne gegenüber der P-Achse verkippt. In der PD-Ebene (Zeichenebene von Figur 2) ist die Projektion der PLA gegenüber der P-Achse entgegen dem Uhrzeigersinne um 3,5° gekippt.

Wie aus der Zeichnung ersichtlich, hat der Schaft 24 einen distalen Schaftabschnitt 30 mit einer abgerundeten Endfläche 32.

Der Schaftabschnitt 30 hat im wesentlichen rechteckigen transversalen Querschnitt, wobei die Schmalseiten des Rechteckes (senkrecht zur Zeichenebene von Figur verlaufend) abgerundet sind und die Längsflächen des Rechteckes jeweils eine flache Vertiefung 34 vorgeben. Die Vertiefung 34 ist somit durch längs der Kanten des Schaftes 24 verlaufende wulstähnliche Rippen 36, 38 begrenzt. Der Querschnitt des distalen Schaftabschnittes 30 nimmt in Proximalrichtung, also in der Zeichnung von unten nach oben kontinuierlich zu. Die Außenfläche des distalen Schaftabschnittes bildet so in etwa eine Pyramide, deren Öffnungwinkel in Dorsalrichtung gesehen (also in der Hauptebene) etwa 10° beträgt. Der Öffnungswinkel in Lateralrichtung gesehen (also senkrecht zur Zeichenebene von Figur 2) beträgt etwa 10°. Ein proximaler Schaftabschnitt 40 des Schaftes 24 verläuft in seinem obersten Endabschnitt parallel zu Achse des Tragzapfens 28. Die Achse des Tragzapfens 28 schließt mit der Proximalachse einen Winkel a ein, der etwa 35° beträgt.

Wie insbesondere aus Figur 2 ersichtlich, ist die Achse des Tragzapfens 28 und des oberen Endabschnittes des proximalen Schaftabschnittes 40 zusätzlich um einen zweiten Winkel b nach ventral aus der durch den distalen Schaftabschnitt 30 vorgegebenen L-P-Ebene herausgekippt. Der Winkel b beträgt beim dargestellten Ausführungsbeispiel etwa 15°.

Die Knicklinie zwischen distalem Schaftabschnitt 30 und proximalem Schaftabschnitt 40 ist beim dargestellten Ausführungsbeispiel um die Strecke 1₁ von etwa 45 mm vom distalen Ende des Schaftes 24 entfernt. Der Abstand 1₂ zwischen der Knicklinie und der Tragplatte 26 beträgt etwa 35 mm.

Der Anstellwinkel c zwischen der Ebene der Tragplatte 26 und der P-Achse (vgl. Figur 1) beträgt 87°. Senkrecht zur PD-Ebene gesehen (Zeichenebene von Figur 2) steht die Tragplatte 26 senkrecht auf dem proximalen Schaftabschnitt 40.

Man kann nun den allermeisten in der Praxis angetroffenen Implantierungsgeometrien dadurch Rechnung tragen, daß man für jede Gesamtlänge des Schaftabschnittes drei Winkel-Varianten und drei Knicklinien-Varianten vorsieht, also insgesamt neun verschiedene Prothesen. Dabei wählt man einen mittleren Knickwinkel von etwa 15° und einen etwas kleineren Knickwinkel von 10° und einen etwas größeren Knickwinkel von 20°. Die Lage der Mittellinie wird vom distalen Ende nach oben bei etwa 60% der Gesamtlänge des Schaftes bzw. 5 mm von dieser Stelle nach distal bzw. proximal versetzt gewählt.

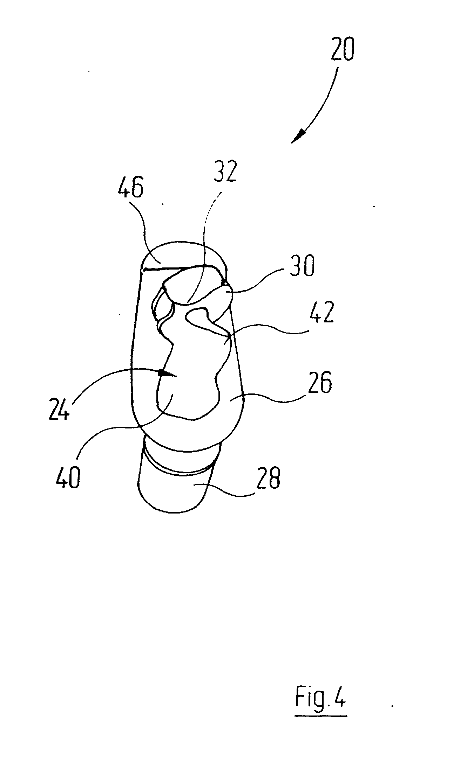

Wie insbesondere aus Figur 4 ersichtlich, hat der distale Schaftabschnitt 30 einen aus seiner Ebene herausgedrehten, tordierten Unterabschnitt 42. In Figur 2 ist Unterabschnitt 44 des proximalen Schaftabschnittes 40 erkennbar, der aus dessen Ebene herausgedreht ist.

Diese tordierten Unterabschnitte bringen eine nochmals bessere Anpassung der Außenfläche des Schaftes 24 an die Innenfläche der Corticalis 14. Der Torsionswinkel des Unterabschnittes 42 um die P-Achse ist etwa 30°, der Torsionswinkel des Unterabschnittes 44 um die Achse des proximalen Schaftabschnittes 40 etwa 15°.

Die Tragplatte 26 hat einen lateralen Seitenabschnitt 46 der zum im Trochander minor liegenden Rand der Corticalis 14 ansteigt. Ein in der Hauptfläche des proximalen Schaftabschnittes liegender Lappen 48, der sich von der distalen Begrenzungsfläche der Tragplatte 26 zur lateralen Seitenfläche des Schaftabschnittes 40 erstreckt, schmiegt sich der Corticalis 14 des oberen Knochenendes an.

Der Prothesenkörper 18 ist durch Schmieden eines Rohlings in einem Gesenk hergestellt. Nach dem Schmieden wurde die Oberfläche durch Sandstrahlen aufgerauht und anschließend geätzt. Auf diese Weise erhält man Mikro-Unebenheiten der Prothesenoberfläche, die scharfkantig sind und an denen sich die Zementschicht 18 gut haftend verkrallen kann.

Die Erfindung wurde obenstehend unter Bezugnahme auf eine zementierte Prothese beschrieben, es versteht sich, daß die im einzelnen angegebenen Geometrien aber analog auch bei zementfrei implantierbaren Prothesen vorgesehen werden können, bei welchen der Schaft eine offene Käfigstruktur bildet.

Es versteht sich ferner, daß die Anwendung der Erfindung nicht auf aus Metall geschmiedete Prothese beschränkt ist, vielmehr auch bei gegossenen Prothesen oder Sinterprothesen verwendbar ist oder auch bei Prothesen die aus anderen Materialien bestehen, z.B. aus Keramik.

## Patentansprüche

1. Prothesenkörper für eine Oberschenkelprothese mit
einem in das obere Ende eines Femurs einsetzbaren Schaft (24), mit einer an dessen oberen Ende angebrachten schräg-transversalen Tragplatte (26) und mit einem von der Tragplatte (26) getragenen nach medial geneigten Tragzapfen (28), auf welchen eine Gelenkkugel (22) aufsetzbar ist, wobei die Länge des Schaftes (24) der Länge des oberen Endabschnittes des Femurs entspricht und der Schaft (24) einen oberen, proximalen Schaftabschnitt (40) und einen unteren, distalen Schaftabschnitt (30) aufweist, wobei die Hauptflächen dieser beiden Schaftabschnitte (24, 40) um einen spitzen Winkel (b) gegeneinander verkippt sind, derart, daß die Schnittlinie zwischen den beiden Hauptflächen der beiden Schaftabschnitte (24, 40) im wesentlichen in Lateralrichtung verläuft, **dadurch gekennzeichnet, daß** die Tragplatte in Umfangsrichtung allseitig im wesentlichen gleichförmig über das obere Ende des Schaftes (24) übersteht.

2. Prothesenkörper nach Anspruch 1, **dadurch gekennzeichnet, daß** der spitze Winkel (b) zwischen 5° und 30°, vorzugsweise zwischen 10° und 20° beträgt.

3. Prothesenkörper nach Anspruch 1 oder 2, **dadurch gekennzeichnet, daß** die Schnittlinie zwischen den Hauptflächen der beiden Schaftabschnitte (24, 30) zwischen 50 und 70 % der Gesamtlänge des Schaftes, vorzugsweise etwa 60% derselben mm vom freien Ende des distalen Schaftabschnittes (30) entfernt ist.

4. Prothesenkörper nach einem der Ansprüchel bis 3,
**dadurch gekennzeichnet, daß** die Tragplatte (26) in einer im wesentlichen senkrecht auf der Proximalachse (P) stehenden Ebene verläuft.

5. Prothesenkörper nach einem der Ansprüche 1 bis 4, **dadurch gekennzeichnet, daß** die Schaftabschnitte (24, 40) unrunden, vorzugsweise im wesentlichen rechteckigen Querschnitt aufweisen, sich in Richtung von proximal nach distal verjüngen und mindestens einer von ihnen einen Unterabschnitt (42, 44) aufweist, der um die Längsachse des betrachteten Schaftabschnittes tordiert ist.

6. Prothesenkörper nach Anspruch 5, **dadurch gekennzeichnet, daß** der Torsionswinkel des Unterabschnittes (42, 44) um die Achse des betrachten Schaftabschnittes (24, 40) zwischen 5 und 30°, vorzugsweise zwischen 10 und 15° beträgt, wobei wiederum vorzugsweise ein Unterabschnitt (44) des proximalen Schaftabschnittes (40) um einen kleineren Winkel tordiert ist als ein Unterabschnitt (42) des distalen Schaftabschnittes (30).

7. Prothesenkörper nach einem der Ansprüche 1 bis 6, **dadurch gekennzeichnet, daß** die Schaftabschnitte (30, 40) im wesentlichen glatt durchgehende Oberfläche aufweisen.

8. Prothesenkörper nach einem der Ansprüche 1 bis 7, **dadurch gekennzeichnet, daß** die Oberflächen der Schaftabschnitte (30, 40) und vorzugsweise auch der Tragplatte (26) z.B. durch Sandstrahlen erzeugte Mikrounebenheiten aufweisen, die vorzugsweise zusätzlich geätzt sind.

9. Prothesenkörper nach einem der Ansprüche 1 bis 8, **dadurch gekennzeichnet, daß** die Tragplatte (26) lateral einen hochgezogenen seitlichen glatten Abschnitt (46) aufweist, dessen Ebene vorzugsweise einen Winkel von 100° bis 150° zum Hauptabschnitt der Tragplatte (26) bildet.

10. Prothesenkörper nach einem der Ansprüche 1 bis 9, **dadurch gekennzeichnet, daß** er ein Schmiedeteil oder Gußteil aus einem biokompatiblen Material, insbesondere aus Titan ist.

## Claims

1. A prosthesis element for a thigh prosthesis with a shaft (24), which can be inserted into the upper end of a femur, with an inclined transverse support plate (26) and with a support post (28) inclined in the medial direction, which is supported by the support plate (26) and onto which a condyle (22) can be fitted, the shaft (24) com- prising an upper, proximal shaft section (40) and a lower, distal shaft section (30), the main surfaces of these two shaft sections (24, 40) being tilted relative to one another through an acute angle (b) in such a manner that the line of intersection between the two main surfaces of the two shaft sections (24, 40) extends substantially in the lateral direction, **characterized in that** the support plate (26) projects beyond the upper end of the shaft (24) on all sides in essentially uniform manner..

2. A prosthesis element as claimed in claim 1, **characterized in that** the acute angle (b) measures between 5° and 30°, preferably between 10° and 20°.

3. A prosthesis element as claimed in claim 1 or 2, **characterized in that** the line of intersection between the main surfaces of the two shaft sections (24, 30) lies at a distance of between 50 and 70% of the overall length of the shaft, preferably approxi-mately 60% of the same from the free end of the distal shaft section (30).

4. A prosthesis element as claimed in one of claims 1 to 3, **characterized in that** the support plate (26) extends in a plane lying substantially perpendicular to the proximal axis (P).

5. A prosthesis element as claimed in one of claims 1 to 4, **characterized in that** the shaft sections (24, 40) comprise a non- circular, preferably substantially rectangular cross section, taper in the direction from proximal to distal and at least one shaft section comprises a lower section (42, 44), which is twisted about the longitudinal axis of the shaft section in question.

6. A prosthesis element as claimed in claim 5, **characterized in that** the angle of torsion of the lower section (42, 44) about the axis of the shaft section (42, 44) in question measures between 5 and 30Þ, preferably between 10 and 15Þ, a lower section (44) of the proximal shaft section (40) again being preferably twisted through a smaller angle than a lower section (42) of the distal shaft section (30).

7. A prosthesis element as claimed in one of claims 1 to 6, **characterized in that** the shaft sections (30, 40) comprise a substantially smooth, continuous surface.

8. A prosthesis element as claimed in one of claims 1 to 7, **characterized in that** the surfaces of the shaft sections (30, 40) and preferably also of the support plate (26) comprise micro- irregularities produced by sandblasting, which are preferably additionally etched.

9. A prosthesis element as claimed in one of claims 1 to 8, **characterized in that** laterally the support plate (26) comprises a raised lateral smooth section (46), whose plane preferably forms an angle of 100° to 150° with the main section of the support plate (26).

10. A prosthesis element as claimed in one of claims 1 to 9, **characterized in that** said element is a forged element or cast element of bicompatible material, more particularly titanium.

## Revendications

1. Corps de prothèse pour une prothèse de cuisse comprenant une tige (24) adaptée d'être placée dans l'extremité supérieure d'un femur, une plaque portante fixée à l'extremité supérieure de cette tige de manière oblique et transversal et comprenant un tenon portant (26) arrangé sur la plaque portante (26) étant inclinée en direction médiale et adaptée de recevoir une sphère de joint (22), la longueur de la tige (24) correspondant à la longueur de la partie supérieure du femur et la tige (24) ayant une partie proximale supérieure (40) ainsi qu'une partie distale inférieure (30), les surfaces principales de ces deux parties de tige (24, 40) étant inclinées entre eux par un angle aigu (b) de manière que la ligne d'intersection entre les deux surfaces principales des deux parties de tige (24, 40) s'étend essentiellement en direction laterale, **caractérisé en ce que** la plaque portante saillit de l'extremité supérieure de la tige (24) dans toutes les directions de manière essentiellement uniforme en direction circonférentielle.

2. Corps de prothèse selon la revendication 1, **caractérisé en ce que** l'angle aigu (b) est compris entre 5° et 30°, par préférance entre 10° et 20°.

3. Corps de prothèse selon la revendication 1 ou 2, **caractérisé en ce que** la ligne d'intersection entre les surfaces principales de deux parties de tige (24, 30) est éloignée de l'extremité libre de la partie distale (30) de la tige d'une distance étant entre 50 et 70 % de la longueur totale de la tige, par préférénce de environs 60 % de la longueur totale de la tige.

4. Corps de prothèse selon une des revendications 1 à 3, **caractérisé en ce que** la plaque portante (26) s'étend dans un plan étant essentiellement perpendiculaire à l'axe proximale (P).

5. Corps de prothèse selon une des revendications 1 à 4, **caractérisé en ce que** les parties de tige (24, 40) ont une section non-circulaire par préférence essentiellement rectangulaire, que ces sections de tige diminuent en direction allandt de proximal vers distal et au moins une d'eux comprend une sous-partie (42, 44) étant tordue par rapport à l'axe longitudinale de la partie de tige considerée.

6. Corps de prothèse selon la revendication 5, **caractérisé en ce que** l'angle de torsion de la sous-partie (42, 44) par rapport à l'axe de la partie de tige (24, 40) considerée est entre 5 et 30°, par préférence entre 10 et 15°, une sous-partie (44) de la partie proximale (40) de la tige étant en outre par préférence tordue par un angle étant plus petit que celui de la sous-partie (42) de la partie distale (30) de la tige.

7. Corps de prothèse selon une des revendications 1 à 6, **caractérisé en ce que** les parties de tige (30, 40) comprennent une surface continue et essentiellement sans discontinuitées.

8. Corps de prothèse selon une des revendications 1 à 7, **caractérisé en ce que** les surfaces des parties de tige (30, 40) et de préférence aussi de la plaque portante (26) montrent des micro-aspérités produites par exemple par l'écapage au sable ces surfaces étant de préférence en plus rongées.

9. Corps de prothèse selon une des revendications 1 à 8, **caractérisé en ce que** la plaque portante (26) dans sa partie laterale est formé avec une partie (46) en saillie laterale et lisse, dont le plan forme, par préférence, un angle de 100° à 150° par rapport à la partie principale de la plaque portante (26).

10. Corps de prothèse selon une des revendications 1 à 9, **caractérisé en ce que** il est une pièce forgée ou une pièce coulée d'un materiaux biocompatible, notamment de titane.
